# EUROPÄISCHE PATENTSCHRIFT

(11) **EP 1 584 301 B1**
(45) Veröffentlichungstag und Bekanntmachung des Hinweises auf die Patenterteilung: **29.11.2006**
(21) Anmeldenummer: 05011114.5
(22) Anmeldetag: 31.05.2000
(51) Int. Cl.: B65D 77/04, B65D 75/58, A61B 19/02, B65D 75/54, B65D 77/02

(54) **Packung zum Lagern von Sterilgut**
Package for a sterile product
Emballage pour le stockage d'un produit stérile

(30) Priorität: 12.07.1999 DE 19932458
(43) Veröffentlichungstag der Anmeldung: 12.10.2005
(62) Teilanmeldung aus: 03000678.7
(73) Patentinhaber: Lohmann & Rauscher GmbH, 2525 Schönau/Triesting (AT)
(72) Erfinder: Waldner, Wilhelm, Ing., 2525 Schönau/Triesting (AT); Leuprecht, Helmut, Dr., 1130 Wien (AT); Koch, Reinhard, 53489 Sinzig (DE)
(74) Vertreter: Leinweber & Zimmermann

(56) Entgegenhaltungen:
- DE-A- 3 638 511
- DE-A- 19 603 476
- DE-A- 19 641 199
- DE-A- 19 737 458
- FR-A- 1 391 971
- US-A- 4 337 862
- US-A- 4 714 595
- US-A- 5 511 358

## Beschreibung

Die Erfindung betrifft eine Packung zum Lagern und Darreichen von Sterilgut mit einer einen sterilen Innenraum aufweisenden Außenverpackung, eine das Sterilgut enthaltenden und im sterilen Innenraum der Außenverpackung aufgenommenen sterilen Innenverpackung und mindestens zwei zumindest den Inhalt der Innenverpackung darstellende Informationen tragenden Informationsträgern, von denen mindestens einer im sterilen Innenraum der Außenverpackung angeordnet ist, wobei mindestens einer der Informationsträger beim Vorgang der Entnahme der Innenverpackung aus der Außenverpackung ohne Kontamination der Innenverpackung von einer unsterilen Person erfaßbar ist, und wobei mindestens einer der Informationsträger ein mit einem den Inhalt der sterilen Innenverpackung angebenden Aufdruck versehenes Klebeetikett aufweist.

Derartige Packungen werden beispielsweise zum Bereitstellen von während einer Operation benötigten Verbandstoffen eingesetzt.

Dazu wird die Außenverpackung üblicherweise von einer unsterilen Person geöffnet. Aus der so geöffneten Außenverpackung entnimmt dann eine sterile Person die Innenverpackung, öffnet diese und entnimmt daraus das Sterilgut, wie etwa die benötigten Verbandstoffe.

Nach Abschluß einer Operation und vor Verschluß der Operationswunde muß durch eine Zählkontrolle sichergestellt werden, daß alle benutzten Verbandstoffe und sonstigen Sterilgüter aus dem Körper des Patienten entfernt worden sind. Zu diesem Zweck sind Packungen der eingangs beschriebenen Art üblicherweise mit zumindest den Inhalt der Packung angebenden Zählkarten ausgestattet. Nach den einschlägigen Vorschriften ist es erforderlich, daß die abschließende Zählkontrolle sowohl von einer sterilen Person im Operationssaal, als auch von einer unsterilen Person außerhalb des Operationssaals durchgeführt wird, bevor die Operationswunde verschlossen werden darf. Aus diesem Grund sind in jeder Packung der eingangs angegebenen Art mindestens zwei Zählkarten enthalten.

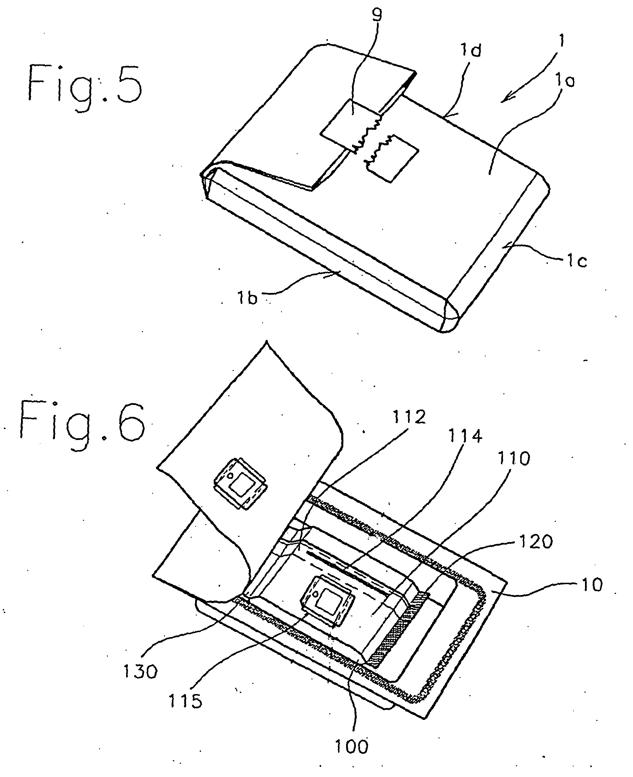

Eine mit diesen beiden Zählkarten ausgestattete Innenverpackung einer Packung der eingangs angegebenen Art nach dem Stand der Technik ist in Fig. 4 dargestellt. Bei dieser Packung ist auf der Außenseite einer in Form eines mit Klebestreifen verschlossenen Papierbeutels 1 verwirklichten und mit Verbandstoffprodukten befüllten Innenverpackung eine zweiteilige Zählkarte mit einem kreisförmigen Klebeelement 2 befestigt. Die Zählkarte selbst besteht aus einer zweiteiligen Klebeetikette 4, 5 mit einem den Inhalt der Innenverpackung darstellenden Aufdruck 8 und einem Trägerstreifen 3 aus nichtklebendem Silikonpapier, auf dem die Klebeetiketten 4, 5 angebracht sind. Die beiden Klebeetiketten 4, 5 und der. Trägerstreifen 3 sind mit einer Perforation 7 versehen. Diese Perforation bildet eine Trennlinie, welche die Trennung der einzelnen Klebeetiketten voneinander ermöglicht. Weiter ist in Fig. 4 erkennbar, daß jede der Klebeetiketten 4, 5 mit einer Lochstanzung 6 versehen ist, die zum Aufhängen der gesammelten Zählkarten dient.

Zur Herstellung einer fertigen Packung wird die in Fig. 4 dargestellte Anordnung in eine Außenverpackung (in Fig. 4 nicht dargestellt) eingelegt und sterilisiert. Zur Bereitstellung der in der in Fig. 4 dargestellten Innenverpackung 1 enthaltenen Verbandstoffprodukte während einer Operation wird zunächst die in Fig. 4 nicht dargestellte Außenverpackung von einer unsterilen Person geöffnet. Danach entnimmt eine sterile Person, wie etwa eine OP-Schwester die Innenverpackung 1. Im weiteren Verlauf nimmt die sterile Person die zweiteilige Zählkarte von der Innenverpackung 1 ab, trennt die Zählkarten 4, 5 längs der Trennlinie 7 voneinander und übergibt eines der Zählkartenteile der unsterilen Person.

Dabei muß darauf geachtet werden, daß sowohl die sterile Person als auch die unsterile Person die zu übergebende Zählkarte jeweils an einem ihrer äußeren Enden anfaßt, um so eine Kontaminierung der sterilen Person zu vermeiden. Nicht verhindert werden kann dabei jedoch, daß die unsterile Person der sterilen Person während der Übergabe sehr nahe kommt. Daher ist die Übergabe der Zählkarte an die unsterile Person mit einer erheblichen Kontaminationsgefahr verbunden, die nur bei einer äußerst sorgfältigen Handhabung auf einem noch akzeptablen Maß gehalten werden kann. Ein weiteres Problem bei der Benutzung von Packungen der in Fig. 4 dargestellten Art ist darin zu sehen, daß beim Nachreichen von Verbandstoffen, was beispielsweise bei einem unerwarteten Operationsverlauf notwendig werden kann, der oben bereits erläuterte Vorgang des Öffnens der Außenverpackung und der Entnahme der Innenverpackung wiederholt werden muß, wobei die sterile Person jedoch bereits durch Blut des zu operierenden Patienten kontaminiert ist. Daher besteht hier die Gefahr, daß die unsterile Person bei der Übergabe der Zählkarte beschmutzt bzw. kontaminiert wird. Ferner steht der unsterilen Person nach Übergabe der Zählkarte nur eine kontaminierte bzw. blutige Zählkarte für die Kontrolle und Dokumentation zur Verfügung. Um das zu verhindern, bedarf es derzeit eines sehr aufwendigen Handlings.

Eine Packung mit zwei den Inhalt einer sterilen Innenverpackung darstellenden Informationsträgern, von denen im sterilen Innenraum einer Außenverpackung angeordnet ist, ist in der FR 1 391 971 A angegeben.

Ferner ist eine Packung, bei der ein Informationsträger im Innenraum der Außenverpackung und ein Informationsträger im Außenraum der Außenverpackung angeordnet ist, in der US 4 714 595 A beschrieben.

Aus der DE 196 41 199 A ist es bekannt, die Klebefläche eines Klebeetikettes mit einer nicht klebenden Abdeckung abzudecken.

Angesichts der vorstehend erläuterten Probleme im Stand der Technik liegt der Erfindung die Aufgabe zugrunde, eine Packung der eingangs beschriebenen Art bereitzustellen, bei deren Benutzung die erforderliche Zählkontrolle ohne Kontaminationsgefahr auf einfache Weise durchgeführt werden kann, und insbesondere eine beschädigungsfreie Abnahme der Klebeetiketten von der Innenverpackung bzw. der Außenverpackung sichergestellt werden kann.

Erfindungsgemäß wird diese Aufgabe dadurch gelöst, daß die Klebefläche mindestens eines der Klebeetiketten zumindest teilweise von einer nicht klebenden Abdeckung abgedeckt ist und mindestens eine der Klebeetiketten einen zum Abtrennen eines zumindest einen Teil der Abdeckung aufweisenden Abschnitts des Klebeetikettes vom Rest des Klebeetikettes dienende Trennlinie aufweist.

Dabei ist die Trennlinie vorzugsweise außerhalb der Abdeckung angebracht.

Zweckmäßig kann die Trennlinie beispielsweise durch eine Perforation gebildet sein.

In besonders vorteilhafter Ausgestaltung der Erfindung weist jede der Klebeetiketten zwei parallel zueinander verlaufende Trennlinien auf, zwischen denen die Abdeckung und der den Inhalt der Innenverpackung angebende Aufdruck angebracht ist. Dann kann die Klebeetikette an zwei Stellen auf die Innenverpackung bzw. auf die Außenverpackung aufgeklebt werden und der zwischen diesen beiden Klebestellen liegende und mit den den Inhalt der Packung angebenden Aufdruck versehene Bereich kann einfach abgenommen werden.

Eine die gewünschte Erfassung durch eine unsterile Person ohne Kontamination der Innenverpackung ermöglichende Anordnung eines Informationsträgers kann besonders einfach verwirklicht werden, wenn dieser Informationsträger an der Außenverpackung befestigt ist.

Folgend wird noch eine Vorrichtung zum Herstellen einer Packung beschrieben. Diese ist mit einer Einrichtung zum Verschließen einer die sterile Innenverpackung enthaltenden Außenverpackung und einem zum Befestigen von Informationsträgern an der sterilen Innenverpackung betreibbare Befestigungseinrichtung ausgestattet und im wesentlichen dadurch gekennzeichnet, daß die Befestigungseinrichtung auch noch zum Befestigen von Informationsträgern an der Außenverpackung betreibbar ist.

Dazu weist die Verschließeinrichtung der Vorrichtung zweckmäßigerweise eine erste zum Fördern der Außenverpackung längs einer vorgegebenen Bahn betreibbare Fördereinrichtung und eine zweite zum Fördern einer Abdeckfolie für die Außenverpackung längs der vorgegebenen Bahn betreibbare Fördereinrichtung auf, wobei die zweite Fördereinrichtung eine zum Umlenken der Abdeckfolie um mindestens 90° vorzugsweise etwa 180° um eine senkrecht zu der vorgegebenen Bahn verlaufende Umlenkachse dienende Umlenkeinrichtung aufweist. Durch diese Ausgestaltung der zweiten Fördereinrichtung wird erreicht, daß die die sterile Innenverpackung enthaltende Außenverpackung, wie etwa eine tiefgezogene Schale vor Anbringen der Abdeckfolie offen und von außen zugänglich ist, um so das Anbringen der Informationsträger an den in die Außenverpackungen eingelegten Innenverpackungen zu ermöglichen. Ferner wird durch diese Anordnung der zweiten Fördereinrichtung erreicht, daß die nach Fertigstellung der Packung dem sterilen Innenraum zugewandte Seite der Abdeckfolie vor der Umlenkung frei zugänglich ist und ebenfalls mit einem Informationsträger ausgestattet werden kann.

Zur vollautomatischen maschinellen Fertigung Packungen weist die Befestigungseinrichtung der Vorrichtung eine zum Zuführen der Informationsträger in einer etwa senkrecht zur vorgegebenen Bahn verlaufenden Zuführrichtung betreibbare Zuführeinrichtung sowie ein Befestigungselement, wie etwa einen in einer senkrecht zu der durch die Zuführrichtung und die vorgegebene Bahn aufgespannten Ebene verlaufenden Aufdruckrichtung hin und her bewegbaren Stempel, auf. Durch einen entsprechend getakteten Betrieb der ersten und zweiten Fördereinrichtung sowie der Zuführeinrichtung und des Befestigungselementes können die erfindungsgemäßen Packungen vollautomatisch hergestellt werden. Dabei kann die Zuführeinrichtung zum Zuführen von auf der Innenverpackung anzubringenden Informationsträgern und auf der der Innenverpackung zugewandten Innenseite der Abdeckfolie anzubringenden Informationsträgern eingesetzt werden, wenn die Zuführeinrichtung und/oder das Befestigungselement in einer etwa parallel zu der vorgegebenen Bahn verlaufenden Richtung hin- und hergehend bewegbar sind. Auf diese Weise kann die Zuführeinrichtung und/oder das Befestigungselement in einer ersten Stellung längs der vorgegebenen Bahn zum Anbringen der Informationsträger auf den in den noch nicht verschlossenen Außenverpackungen aufgenommen Innenverpackungen und in einer durch Verschieben längs der vorgegebenen Bahn erreichten zweiten Stellung zum befestigen von Informationsträgern auf der Abdeckfolie vor deren Umlenkung benutzt werden.

Ein besonders störungsfreier Betrieb der Vorrichtung zum Befestigen von in der erfindungsgemäßem Form von Klebeetiketten hergestellten Informationsträgern auf der Innenverpackung und der Außenverpackung kann erreicht werden, wenn die Zuführeinrichtung zum Zuführen von auf einer nichtklebenden Trägerfolie, wie etwa Silikonpapier angebrachten Klebeetiketten ausgelegt ist und mindestens eine Abzieheinrichtung zum Abziehen mindestens eines etwa parallel zur Zuführrichtung verlaufenden Randbereichs der Trägerfolie von den Klebeetiketten umfaßt. Nach Abziehen dieses Randbereiches können die Klebeetiketten dann mit ihrer so freigelegten Klebefläche an der Innenverpackung bzw. der Abdeckfolie der Außenverpackung befestigt werden. Wie vorstehend bereits erläutert, ist es besonders zweckmäßig, wenn die Klebeetiketten mit zwei parallel zueinander verlaufenden Klebeflächen auf der Innenverpackung bzw. der Abdeckfolie der Außenverpackung befestigt sind, wobei ein mit einem den Inhalt der Packung angebenden Aufdruck versehener Bereich der Klebeetiketten zwischen diesen freiliegenden Klebestreifen angeordnet ist und auf seiner dem Aufdruck abgewandten Seite eine noch mit der Abdeckfolie abgedeckte Klebefläche umfaßt.

Mit der Vorrichtung kann eine besonders hohe Produktionsgeschwindigkeit erreicht werden, wenn die Befestigungseinrichtung zum gleichzeitigen Festlegen von mindestens zwei Informationsträgern auf mindestens zwei Innenverpackungen und/oder mindestens zwei Außenverpackungen betreibbar ist und/oder die erste Fördereinrichtung zum Fördern von mindestens zwei in einer senkrecht zu der vorgegebenen Bahn verlaufenden Richtung nebeneinander angeordneten Außenverpackungen ausgelegt ist.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- Fig. 1: eine perspektivische Ansicht einer erfindungsgemäßen Packung
- Fig. 2: eine schematische Seitenansicht einer Vorrichtung zum Herstellen der in Fig. 1 dargestellten Packung,
- Fig. 3: eine schematische Draufsicht auf die in Fig. 2 dargestellte Vorrichtung,
- Fig. 4: eine perspektivische Ansicht einer Innenverpackung einer Packung zum Lagern von Sterilgut nach dem Stand der Technik,
- Fig. 5: eine perspektivische Ansicht einer zum Herstellen einer erfindungsgemäßen Packung geeigneten herkömmlichen Innenverpackung,
- Fig. 6: eine perspektivische Ansicht einer in einer Außenverpackung aufgenommenen Innenverpackung,

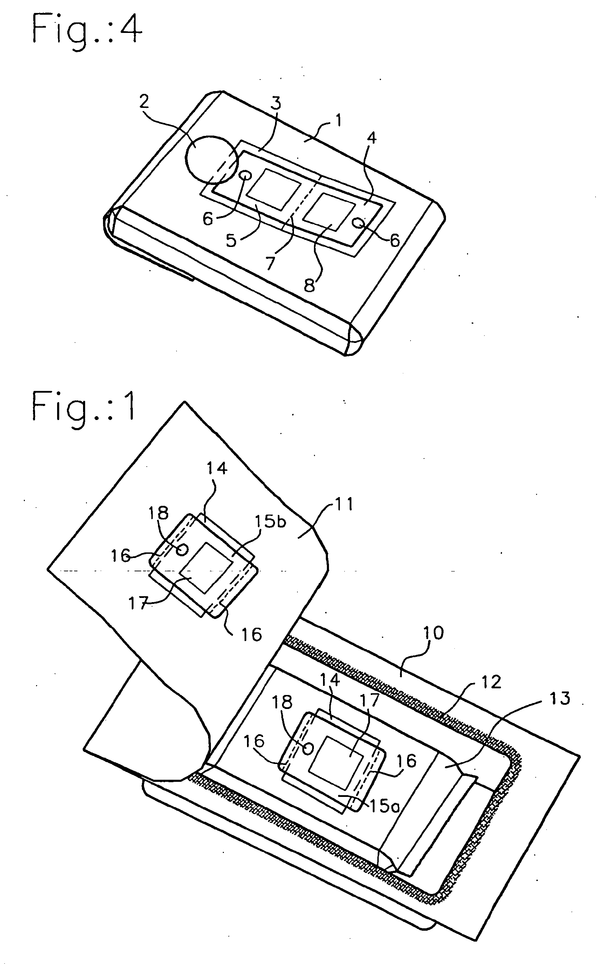

Die in Fig. 1 dargestellte Packung umfaßt eine Innenverpackung 13 in Form eines maschinell befüllten und verschlossenen Papierschlauchbeutels 13 sowie eine in Form einer tiefgezogenen Schale 10 mit einer aufgesiegelten Papierdeckbahn 11 gebildete Außenverpackung. Bei der Herstellung dieser Packungen wird der Papierschlauchbeutel-13-in einer Verpackungsmaschine in die tiefgezogene Schale 10 eingelegt. Vor dem Aufsiegeln der Papierdeckbahn 11 werden Zählkarten getrennt voneinander einerseits auf den Papierschlauchbeutel 13 und andererseits an der dem Innenraum der tiefgezogenen Schale 10 zugewandten Seite der Papierdeckbahn 11 angebracht.

Dabei umfaßt jede der Zählkarten ein mit einem Aufdruck 17 versehenes Klebeetikett 15a und 15b, deren dem Papierschlauchbeutel 13 bzw. der Innenseite der Papierdeckbahn 11 zugewandte Klebefläche teilweise von einer Trägerfolie 14 abgedeckt ist. Jede der Klebeetiketten 15a und 15b umfaßt jedoch parallel zueinander verlaufende und beidseits der nichtklebenden Trägerfolie angeordnete Klebeflächen, von denen jede mit einer parallel zu einem Rand der Trägerfolie 14 verlaufenden Perforation 16 versehen ist. Mit diesen Klebeflächen können die Klebeetiketten auf dem Papierschlauchbeutel 13 bzw. die Innenseite der Papierdeckbahn 11 aufgeklebt werden, während die Perforationen 16 ein einfaches, sicheres und beschädigungsfreies Abnehmen der Klebeetiketten 15a und 15b von dem Papierschlauchbeutel 13 bzw. der Papierdeckbahn 11 ermöglichen.

Wie in Fig. 1 weiter dargestellt, weist jede der Klebeetiketten bzw. Zählkarten 15a und 15b auch noch eine Lochstanzung 18 auf, die zum Aufhängen der gesammelten Zählkarten eingesetzt werden kann.

Beim Einsatz der in Fig. 1 dargestellten Packung wird zunächst die aus der tiefgezogenen Schale 10 und Papierdeckbahn 11 bestehende Außenverpackung durch eine unsterile Person geöffnet, in dem die Papierdeckbahn von der tiefgezogenen Schale abgezogen wird. Dieser Zustand ist in Fig. 1 dargestellt. Danach entnimmt eine sterile OP-Schwester den das Sterilgut, wie etwa Verbandstoffprodukte, enthaltenden Papierschlauchbeutel 13. Die OP-Schwester kann dann die Zählkarte bzw. das Klebeetikett von dem Papierschlauchbeutel 13 abnehmen und den Beutelinhalt überprüfen. Andererseits kann die auf der Innenseite der Papierdeckbahn 11 angebrachte Zählkarte 15b ohne Gefahr einer Kontamination des Papierschlauchbeutels 13 von der unsterilen Person von der Innenseite der Papierdeckbahn 11 abgenommen werden, die die Außenverpackung sowieso in den Händen hält. Dadurch wird eine Kontaminationsgefahr sicher ausgeschaltet.

Ein entscheidender Vorteil der gerade erläuterten erfindungsgemäßen Packung ist also darin zu sehen, daß aufgrund der getrennt angebrachten Zählkarten 15 und 15b die sterile und die unsterile Person nicht mehr, wie bei der alten Verpackungsweise mit den Händen nahe zusammenkommen müssen. Dadurch wird die Kontaminationsgefahr über die Zählkarten ausgeschaltet und eine Voraussetzung für eine kontaminationsfreie Dokumentation gewährleistet.

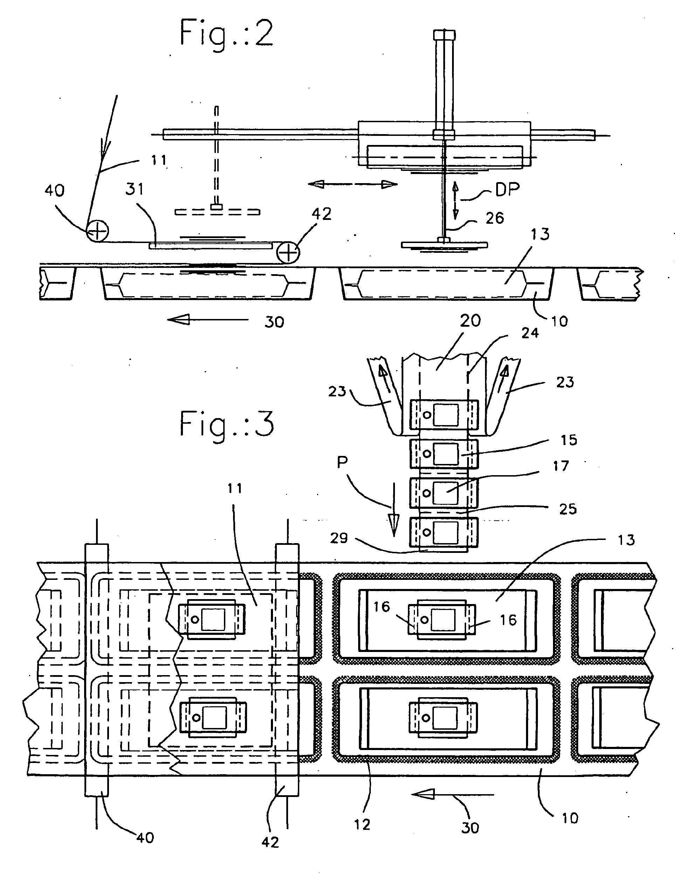

In den Fig. 2 und 3 ist die Anbringung der Zählkarten auf der in Fig. 1 dargestellten Packung vereinfacht dargestellt.

Dazu werden mit den benötigten Informationen, wie etwa dem Packungsinhalt, der Menge, dem Verfallsdatum, der Chargenbezeichnung, notwendigen Prüfzeichen u. dgl., bedruckte Klebeetiketten 15, die auf einer Trägerfolie 20 aus Silikonfolie oder einer anderen nichtklebenden Substanz angebracht sind, benutzt. Dabei sind die Klebeetiketten bereits in einer gewünschten Anordnung positioniert. Der aus der Trägerfolie und den Klebeetiketten bestehende Streifen wird dann getaktet durch eine Zuführeinrichtung in einer durch den Pfeil P bezeichneten Zuführrichtung zugeführt. Gleichzeitig werden während des Zuführvorgangs zwei parallel zur Zuführrichtung P verlaufende Randstreifen 23 der Trägerfolie 20 mit Hilfe einer bereits vorhandenen Perforation 24 abgetrennt. Die danach verbleibende Trägerfolie 29 ist in einer senkrecht zur Zuführrichtung P verlaufenden Richtung schmaler als die Klebeetiketten 15, so daß eine selbstklebende Unterseite der Klebeetiketten 15 beidseitig über die Trägerfolie 29 hinausragt. Die einzelnen Klebeetiketten 15 sind in der Zuführrichtung P voneinander beabstandet. Dadurch ergibt sich ein Überstand der Trägerfolie in der Zuführrichtung P.

Die aus der Trägerfolie 29 und den Klebeetiketten 15 bestehenden Zählkarten werden nun mittels einer in der Zeichnung nicht näher dargestellten Fördereinrichtung über den mit Hilfe einer ersten Fördereinrichtung in einer senkrecht zur Zuführrichtung P verlaufenden und durch den Pfeil 30 bezeichneten Richtung geförderten tiefgezogenen Folien 10 positioniert und durch einen Stempel 26 (vgl. Fig. 2) auf bereits in den tiefgezogenen Schalen 10 angeordneten Papierschlauchbeuteln befestigt. Dazu ist der Stempel 26, wie in Fig. 2 durch den Doppelpfeil DP angedeutet in einer senkrecht zur Förderrichtung der ersten Fördereinrichtung und der Zuführrichtung aufgespannten Ebene verlaufenden Richtung hin- und hergehend bewegbar. Im weiteren Verlauf der Förderung der tiefgezogenen Schalen 10 längs der durch den Pfeil 30 angegebenen vorgegebenen Bahn wird die Öffnung der einzelnen tiefgezogenen Schalen mit einer Papierdeckbahn 11 verschlossen.

Die Papierdeckbahn 11 wird mit einer eine erste Umlenkrolle 40 und eine zweite Umlenkrolle 42 aufweisenden Fördereinrichtung derart zugeführt, daß sie zwischen der ersten Umlenkrolle 40 und der zweiten Umlenkrolle 42 in einer der Förderrichtung 30 der ersten Fördereinrichtung entgegengestetzten Richtung gefördert wird und erst nach Umlaufen der zweiten Umlenkrolle 42 parallel zur Förderrichtung 30 der ersten Fördereinrichtung gefördert wird und auf die tiefgezogene Schale 10 aufgesiegelt werden kann.

In dem zwischen der ersten Umlenkrolle 40 und der zweiten Umlenkrolle 42 liegenden Streckenabschnitt werden die Klebeetiketten auf der im fertiggestellten Zustand dem Innenraum der tiefgezogenen Schale 10 zugewandten Innenseite der Papierdeckbahn 11 angebracht. Dazu ist in dem Zwischenraum zwischen der Papierdeckbahn 11 und den längs der vorgegebenen Bahn geförderten tiefgezogenen Schale im Bereich zwischen der ersten Umlenkrolle 40 und der zweiten Umlenkrolle 42 eine Gegenplatte 31 angebracht, mit der eine Beschädigung der Papierdeckbahn 11 während des Anbringens der Klebeetiketten darauf verhindert werden kann. Wie in Fig. 2 durch den Doppelpfeil 44 angedeutet, ist die zum Zuführen der Klebeetiketten ausgelegte Zuführeinrichtung und der Stempel 26 längs der vorgegebenen Bahn der tiefgezogenen Schalen 10 hin- und hergehend bewegbar. Dadurch wird erreicht, daß die in Fig. 3 dargestellte Befestigungseinrichtung in einer ersten in Fig. 2 mit durchgezogenen Linien dargestellten Position zum Anbringen von Klebeetiketten an den Papierschlauchbeuteln 13 und in einer zweiten in Fig. 2 strichliert dargestellten Position zum Anbringen der Klebeetiketten auf der Innenseite der Papierdeckbahn 11 betrieben werden kann.

Wie besonders deutlich der Fig. 3 zu entnehmen ist, sind die einzelnen Perforationen 16 beidseits, außerhalb und parallel zur Trägerfolie angeordnet. Dadurch kann die nur an den Rändern aufgeklebte Zählkarte leicht mit zwei Fingern gefaßt und von der Packung gelöst werden.

Weiter ist der Fig. 3 zu entnehmen, daß die erfindungsgemäße Vorrichtung zur Fertigstellung von zwei oder mehr parallel zueinander geförderten Packungen ausgelegt sein kann.

Fig. 6 zeigt eine in einer in Form einer tiefgezogenen Schale 10 ausgeführten Außenverpackung aufgenommene Innenverpackung. Diese Innenverpackung weist eine in Form eines Schlauchbeutels 100 aus Papier gebildete Hülle auf. Diese Hülle 100 bildet eine in Fig. 6 nach oben weisende, im wesentlichen recheckige Hauptfläche 110 auf der ein Informationsträger 115 in der anhand der Fig. 1 erläuterten Weise angebracht ist. Die den Schlauchbeutel 100 bildende Materialbahn weist zwei in Längsrichtung verlaufende Ränder auf, welche sich im Bereich der Hauptfläche 110 überlappen und längs einer Verbindungslinie 114 miteinander verbunden sind. Längs ihrer quer zu der Verbindungslinie 114 bzw. den Längsrändern verlaufenden Querränder sind die einander gegenüberliegenden Abschnitte der den Schlauchbeutel 100 bildenden Materialbahn miteinander versiegelt.

## Patentansprüche

1. Packung zum Lagern von Sterilgut mit einer einen sterilen Innenraum aufweisenden Außenverpackung (10, 11), einer das Sterilgut enthaltenden und im sterilen Innenraum der Außenverpackung (10, 11) aufgenommenen sterilen Innenverpackung (13) und mindestens zwei zumindest den Inhalt der Innenverpackung (13) darstellende Informationen tragenden Informationsträgern (15a, 15b), von denen mindestens einer (15a) im sterilen Innenraum der Außenverpackung (10, 11) angeordnet ist, wobei mindestens einer der Informationsträger (15b) beim Vorgang der Entnahme der Innenverpackung (13) aus der Außenverpackung (10, 11) ohne Kontamination der Innenverpackung (13) von einer unsterilen Person erfaßbar ist, und wobei mindestens einer der Informationsträger (15a, 15b) ein mit einem den Inhalt der sterilen Innenverpackung (13) angebenden Aufdruck (17) versehenes Klebeetikett aufweist, **dadurch gekennzeichnet, daß** die Klebefläche mindestens eines der Klebeetiketten zumindest teilweise von einer nichtklebenden Abdeckung (14) abgedeckt ist und mindestens eine der Klebeetiketten (15a, 15b) eine zum Abtrennen eines zumindest einen Teil der Abdeckung aufweisenden Abschnitts des Klebeetikettes vom Rest des Klebeetikettes dienende Trennlinie aufweist.

2. Packung nach Anspruch 1, **dadurch gekennzeichnet, daß** die Trennlinie außerhalb der Abdeckung (14) angebracht ist.

3. Packung nach Anspruch 1 oder 2, **dadurch gekennzeichnet, daß** die Trennlinie durch eine Perforation (16) gebildet ist.

4. Packung nach einem der vorhergehenden Ansprüche, **dadurch gekennzeichnet, daß** mindestens einer der Informationsträger (15b) an der Außenverpackung (11) befestigt ist.

## Claims

1. A package for storing sterile product with an outer packing (10, 11) exhibiting a sterile interior, a sterile inner packing (13) containing the sterile product and accommodated in the sterile interior of the outer packing (10, 11), and at least two information carriers (15a, 15b) carrying at least information representing the content of the inner packing (13), at least one (15a) of which information carriers is arranged in the sterile interior of the outer packing (10, 11), wherein at least one of the information carriers (15b) can be gripped by a non-sterile person during the process of removing the inner packing (13) from the outer packing (10, 11) without contaminating the inner packing (13), and wherein at least one of the information carriers (15a, 15b) exhibits an adhesive label provided with an imprint (17) indicating the content of the sterile inner packing (13), **characterised in that** the adhesive surface of at least one of the adhesive labels is at least partially covered by a non-adhesive cover (14), and **in that** one of the adhesive labels (15a, 15b) exhibits a parting line serving to separate a section of the adhesive label exhibiting at least part of the cover from the rest of the adhesive label.

2. The package according to Claim 1, **characterised in that** the parting line is arranged outside the cover (14).

3. The package according to Claim 1 or 2, **characterised in that** the parting line is formed by a perforation (16).

4. the package according to one of the preceding claims, **characterised in that** at least one of the information carriers (15b) is fastened to the outer packing (11).

## Revendications

1. Emballage pour le stockage d'un produit stérile comprenant un emballage extérieur (10, 11) pourvu d'un compartiment intérieur stérile, un emballage intérieur (13) stérile qui contient le produit stérile et est disposé dans le compartiment intérieur stérile de l'emballage extérieur (10, 11) et au moins deux supports d'information (15a, 15b) qui portent des informations représentant le contenu de l'emballage intérieur (13) et parmi lesquels l'un au moins (15a) est disposé dans le compartiment intérieur stérile de l'emballage extérieur (10, 11), au moins un des supports d'information (15b), lors du processus d'extraction de l'emballage intérieur (13) de l'emballage extérieur (10, 11), pouvant être saisi par une personne non stérile sans risque de contamination de l'emballage intérieur (13) et au moins un des supports d'information (15a, 15b) comportant une étiquette adhésive avec des inscriptions (17) indiquant le contenu de l'emballage intérieur (13) stérile, **caractérisé par le fait que** la surface adhésive d'au moins une des étiquettes adhésives est recouverte au moins partiellement d'une protection (14) non adhésive, et qu'une des étiquettes adhésives (15a, 15b) présente une ligne de séparation pour la séparation du reste de l'étiquette d'une portion de l'étiquette adhésive qui porte au moins une partie de la protection.

2. Emballage selon la revendication 1, **caractérisé par le fait que** la ligne de séparation est disposée en dehors de la protection (14)

3. Emballage selon la revendication 1 ou 2, **caractérisé par le fait que** la ligne de séparation est formée par une perforation (16).

4. Emballage selon une des revendications précédentes, **caractérisé par le fait qu'**au moins un des support d'information (15b) est fixé à l'emballage extérieur (11).
